# EUROPÄISCHE PATENTANMELDUNG

(11) **EP 1 832 278 A1**
(43) Veröffentlichungstag der Anmeldung: **12.09.2007**
(21) Anmeldenummer: 07004947.3
(22) Anmeldetag: 09.03.2007
(51) Int. Cl.: A61K 9/00, A61P 11/02, A61K 33/30, A61K 31/315

(54) **Zinccysteinat zur Behandlung von Entzündungen oder Allergien der Atemwege**

(30) Priorität: 09.03.2006 DE 102006011012
(71) Anmelder: Geske, Gundula, 73277 Owen (DE)
(72) Erfinder: Geske, Gundula, 73277 Owen (DE)
(74) Vertreter: Dey, Michael

(57) **Zusammenfassung**

Die vorliegende Erfindung betrifft die Verwendung von Zinkcysteinat zur Herstellung einer pharmazeutischen Zusammensetzung zur Behandlung von Entzündungen oder Allergien der Atemwege, sowie ein Medizinprodukt umfassend Zinkcysteinat als Wirkstoff, welches als Spray formuliert ist.

## Beschreibung

Die vorliegende Erfindung betrifft die Verwendung von Zinkcysteinat zur Herstellung einer pharmazeutischen Zusammensetzung zur Behandlung von Entzündungen oder Allergien der Atemwege, sowie ein Medizinprodukt umfassend Zinkcysteinat als Wirkstoff, welches als Spray formuliert ist.

In einer Vielzahl an Situationen ist eine rasche und effiziente Aktivierung der T-Zellen des Immunsystems erforderlich, um die Schwere von Erkrankungen zu reduzieren. Dies gilt insbesondere für durch Viren hervorgerufene Erkrankungen der Schleimhäute, welche die primären Eingangspforten für derartige Erreger darstellen. Viren verfügen über vielfältige Mechanismen, welche es ihnen gestatten, die Abwehrfunktionen des Immunsystems zu unterlaufen. Einer dieser Mechanismen besteht in der Produktion funktioneller Analoga immunoregulativer Botenstoffe wie beispielsweise Interleukin 10 (Müller et al., Eur. J. Immunol. 1998, 28, 3488-3498; Dumoutier et al., Eur. Cytokine Netw. 2002, 13, 5). Interleukin 10 (IL-10) besitzt die bei einer Infektion für das Immunsystem nachteilige Eigenschaft, insbesondere Makrophagen in ihrer Aktivität zu drosseln (Keystone et al., Rheum. Dis. Clin. North Am. 1998, 24, 629-639). In Anbetracht der Tatsache, dass die Makrophagen jene Zellen des Immunsystems darstellen, welche die Basis für eine Aktivierung des adaptiven Immunsystems bilden, hat die Produktion von IL-10 und anderer immunoregulativer Botenstoffe durch die Viren massive Auswirkungen auf die Effektivität der Immunantwort von T-Zellen. Da Abwehrreaktionen des Immunsystems im Falle von Erkrankungen der oberen Atemwege, welche in mehr als 90% durch Viren verursacht werden, somit in der Regel nicht von den Fresszellen (Granulozyten und Makrophagen) übernommen werden können, erfordert insbesondere die Abwehr von Erregern in den Schleimhäuten voll funktionsfähige T-Zellen.

Zink zählt zu den essenziellen Spurenelementen für den Stoffwechsel von Lebewesen. Das Metall stellt den Cofaktor zahlreicher Enzymsysteme dar und spielt eine wesentliche Rolle im Vitamin A-, im Kohlenhydrat- sowie im Lipidstoffwechsel. Ferner ist Zink essenziell für die Funktion verschiedener Hormone, wie beispielsweise Insulin, den Schilddrüsenhormonen, Sexualhormonen sowie Wachstumshormonen. Auch im Stoffwechsel von Nukleinsäuren und Proteinen erfüllt Zink wichtige Aufgaben. So dient es beispielsweise der Stabilisierung der DNA- und RNA-Struktur, ist jedoch gleichzeitig ein Bestandteil von Schlüsselenzymen der Nukleinsäuresynthese (z.B. DNA- bzw. RNA-Polymerasen). Neben einer antiviralen (Korant et al., Nature 1978, 248, 588-590; Merluzzi et al., Res. Comm. Chem. Patol. and Pharmacol. 1989, 66, 425-440) und adstringierenden Wirkung (Osol, in Remington's Pharmaceutical Sciences, 16. Ausgabe, Mack Publishing Co., Easton, 1980, 720-723) konnte für Zink auch eine abschwächende Wirkung in Bezug auf die Symptome von Allergien gezeigt werden (Marone et al., J. Pharmacol. Exp. Ther. 1981, 217, 292-298). Darüber hinaus ist das Metall am Zellwachstum und an der Zelldifferenzierung beteiligt, und wird für eine funktionierende Immunabwehr benötigt. So besteht eine wesentliche Aufgabe von Zink in einer Verbesserung der Funktion von T-Zellen (Mathe et al., Med. Oncol. Tumor Pharmacother. 1985, 2, 203-210; Reardon et al., Immunobiol. 1987, 175, 455-469; Salas et al., Clin. Immunol. Immunopathol. 1987, 45, 139-142).

Der Körper eines erwachsenen Menschen enthält eine Gesamtmenge von etwa 2 g an Zink, welches überwiegend in den Muskeln sowie in den Knochen gespeichert wird. Die absolute Konzentration an Zink kann in einzelnen Gewebearten, in welchen das Metall für bestimmte biochemische Vorgänge benötigt wird, jedoch deutlich höher liegen. Besonders hohe Konzentrationen an Zink befinden sich in den roten Blutkörperchen, in den Augen, in der Haut, in den Haaren, in der Prostata sowie in der Leber. Der tägliche Bedarf an Zink liegt für Erwachsene und Jugendliche bei etwa 7-10 mg. Ein erhöhter Bedarf an Zink kann beispielsweise im Falle bestimmter Erkrankungen, wie etwa Immunschwäche, Krebs, Lebererkrankungen, Diabetes mellitus oder anderen Hauterkrankungen, bei der Einnahme bestimmter Arzneimittel, in der Schwangerschaft und Stillzeit, im Wachstum, sowie bei Leistungssportlern bestehen. Da Zink allerdings im Körper nicht gespeichert werden kann, ist eine regelmäßige Zufuhr des Spurenelements von außen erforderlich.

Die Resorption von Zink kann durch eine Reihe organischer Verbindungen bzw. Komplexbildner, wie beispielsweise Citrat, Glutamat, Histidin oder Cystein erhöht werden. So offenbart beispielsweise DE 2 209 616 Komplexsalze von Cystein bzw. Cysteinestern mit Zink, sowie deren Verwendung als Wirkstoffe in pharmazeutischen Zubereitungen, welche zur Behandlung von Gewebewachstumsstörungen und dermatologischen Erkrankungen verwendet werden können.

US 5,401,770 betrifft antipruritische Mittel, welche Komplexverbindungen von Zink mit verschiedenen Aminosäuren umfassen. Angeführt wird in diesem Zusammenhang auch eine Zusammensetzung, welche eine Zink-Cystein-Komplexverbindung in einer Menge von 3 Gew.-% enthält.

US 6,586,611 B1 beschreibt ein Verfahren zur Bereitstellung von Zink, welches die Verabreichung von Zink-Cystein-Komplexverbindungen, wie beispielsweise Zink-Monocystein-Komplex, vorsieht.

EP 0 957 890 B1 offenbart Zusammensetzungen, welche eine verbesserte Abgabe von Aminosäuren an das Haar und/oder die Kopfhaut bereitstellen. Wie im Rahmen der Druckschrift ausgeführt, umfassen die Zusammensetzungen einen Metall-Aminosäure-Komplex, mindestens ein Tensid und einen Abscheidungshilfsstoff, wobei als Metall-Aminosäure-Komplex bevorzugt Zinkcysteinat eingesetzt wird.

Ferner ist eine Kombination von Zink und Histidin als Curazink^{®}, eine Kombination von Zink, Histidin und Cystein als "tetesept^{®} Zink + Histidin + Cystein" in Form von Kapseln im Handel erhältlich. Dem Cystein wird dabei eine radikalfangende Wirkung zugesprochen. Die Zuführung von Radikalfängern im Verlaufe von Infekten ist jedoch grundsätzlich als bedenklich einzustufen.

Es war daher eine Aufgabe der vorliegenden Erfindung, eine Verabreichungsform für Zink bereitzustellen, welche für die Behandlung von Entzündungen oder Allergien der Atemwege geeignet ist, indem sie eine rasche und effektive Resorption des Spurenelements gestattet.

Diese Aufgabe wird erfindungsgemäß gelöst durch die Verwendung von Zinkcysteinat, welches als pharmazeutische Zusammensetzung in geeigneter Formulierung appliziert wird.

Die vorliegende Erfindung betrifft die Verwendung von Zinkcysteinat zur Herstellung einer pharmazeutischen Zusammensetzung zur Behandlung von Entzündungen oder Allergien der Atemwege.

Die Erfindung betrifft weiterhin ein Medizinprodukt umfassend Zinkcysteinat als Wirkstoff, welches als Spray formuliert ist.

Die Erfindung nutzt die Erkenntnis, dass Cystein neben einer Verbesserung der Bioverfügbarkeit auch hinsichtlich der Aktivierung von T-Zellen eine wichtige Rolle zukommt. Cystein stellt einen verstärkenden Faktor der T-Zellaktivierung dar, dessen Mangel zu einer eingeschränkten Aktivität der T-Zellen führt (Gmünder et al., Cell Immunol. 1990, 129, 32-46; Dröge et al., Klin. Wochenschr. 1991, 69, 1118-1122; Dröge et al., Am. J. Med. 1991, 91 (3C), 140S-144S). Da den T-Zellen sowohl das Enzym Cystathionase, welches die Bildung von Cystein aus der Vorstufe Cystin katalysiert, als auch ein Transporter, welcher die Aufnahme von Cystein aus der Umgebung gestatten würde, fehlt, sind T-Zellen hinsichtlich der Bereitstellung von Cystein auf andere Quellen angewiesen (Eagle et al., Proc. Natl. Acad. Sci. 1966, 56, 156-163; Bannai, Biochim. Biophys. Acta 1984, 779, 289-306; Gmünder et al., Eur. J. Biochem. 1991, 201, 113-117). Die Bereitstellung von Cystein wird von den Makrophagen übernommen, welche über das Enzym Cystathionase verfügen und stets in direkter Nachbarschaft zu den T-Zellen in den Geweben angesiedelt sind. Somit obliegt es den Makrophagen, durch Regulierung der Produktion von Cystein den Grad der Aktivierung von T-Zellen zu steuern (Sido et al., J. Exp. Med. 2000, 192, 907-912). Im Falle der Schleimhäute, wo auf Seiten des adaptiven Immunsystems gamma/delta-positive T-Zellen betroffen sind, erfolgt eine zusätzliche Verknappung des Cysteins durch die Makrophagen.

In Abwesenheit von Cystein kann auch eine Aktivierung der T-Zellen durch Zink nicht in gewünschter Weise erfolgen. Auf Basis der für gamma/delta-positive T-Zellen erhaltenen Ergebnisse ist vielmehr davon auszugehen, dass insbesondere im Bereich der Schleimhäute mögliche Effekte des Zinks ohne gleichzeitig anwesendes Cystein zu einem großen Teil wirkungslos verpuffen. Dies wird durch die erfindungsgemäße Verabreichung von Zinkcysteinat vermieden.

Erfindungsgemäß wird somit insbesondere die immunmodulierende Aktivität von Zink in Kombination mit der T-Zellen-aktivierenden Wirkung von Cystein genutzt.

Die erfindungsgemäßen pharmazeutischen Zusammensetzungen sind bevorzugt als Spray, insbesondere als Nasenspray formuliert. Die Zubereitung als lokal anzuwendendes Spray verhindert, dass die Inhaltsstoffe entweder nur in sehr verdünntem Maße, oder aber infolge Metabolisierung unter Umständen gar nicht an den eigentlichen Wirkort gelangen. Da es sich bei den wirksamen Inhaltsstoffen jeweils um niedermolekulare Stoffe handelt, welche von den Schleimhäuten, insbesondere jedoch von erkrankten Schleimhäuten, bereitwillig resorbiert werden, ist eine Applikation von Zinkcysteinat in Form von Sprays besonders bevorzugt. Die erfindungsgemäßen Zusammensetzungen können jedoch auch in Form von Aerosolen, wässrigen oder nicht-wässrigen Lösungen, Schäumen, Emulsionen, Suspensionen, oder anderen geeigneten Formulierungen appliziert werden.

Die Verabreichung der erfindungsgemäßen Zusammensetzungen erfolgt bevorzugt nasal und/oder oral. Andere Verabreichungsformen, welche für eine Applikation von Zusammensetzungen im Sinne der vorliegenden Erfindung geeignet sind, sind einem Fachmann auf dem Gebiet der Pharmakologie bzw. Medizin bekannt und umfassen, sind jedoch nicht beschränkt auf, nasopharyngeal, inhalativ oder topisch.

Die erfindungsgemäßen Zusammensetzungen werden derart verabreicht, dass pro Verabreichungsdosis bevorzugt eine Menge im Bereich von 2 mg bis 15 mg, und besonders bevorzugt eine Menge im Bereich zwischen 7 mg bis 10 mg an Zink bereitgestellt wird. In einer bevorzugten Ausführungsform handelt es sich bei der verabreichten Menge um eine pharmazeutisch wirksame Menge. Die Konzentration an Zink, bezogen auf die Gesamtformulierung, beträgt insbesondere 0.1-1.5 mM, bevorzugt 0.6-1.0 mM.

Erfindungsgemäß enthalten die pharmazeutischen Zusammensetzungen Zinkcysteinat bevorzugt in Kombination mit einem oder mehreren pharmazeutisch annehmbaren Trägern, Verdünnungsmitteln und/oder Additiven. Der hier verwendete Ausdruck "pharmazeutisch annehmbarer Träger" bezeichnet einen oder mehrere flüssige, halbfeste oder feste Füllstoffe, Verdünnungsmittel oder andere Substanzen, welche für eine Verabreichung an Säuger, einschließlich Menschen, geeignet sind.

Der Ausdruck "Träger" im Sinne der vorliegenden Erfindung bezeichnet dabei jeglichen organischen oder anorganischen, natürlichen oder synthetischen Stoff, welcher zur Vereinfachung der Applikation mit dem Wirkstoff kombiniert werden kann. Beispiele für derartige Träger umfassen, sind jedoch nicht beschränkt auf, organische oder anorganische Lösungsmittel, Stärke, Laktose, Mannitol, Methylcellulose, Talk, Gelatine, Agar-Agar, Calciumphosphat, Magnesiumstearat, tierische und pflanzliche Fette, höhermolekulare Fettsäuren, oder höhermolekulare Polymere.

Der Ausdruck "pharmazeutisch annehmbar" bezeichnet jegliches nichttoxische Material, welches die Wirksamkeit der biologischen Aktivität des Wirkstoffes nicht beeinträchtigt. Derartige Materialien können pharmazeutisch annehmbare Konzentrationen an Salzen, Puffern, Konservierungsstoffen, oder dergleichen umfassen.

Bevorzugte pharmazeutisch annehmbare Träger im Sinne der vorliegenden Erfindung sind flüssige Träger, wie etwa Wasser, wässrige Salzlösungen, nicht-wässrige (wasserfreie) Lösungsmittel, oder Mischungen hieraus. Geeignete nicht-wässrige Lösungsmittel umfassen, sind jedoch nicht beschränkt auf, Ethanol, Propanol, Isopropanol, Butanol, Benzylalkohol, Glycerin, Propylenglykol, Di- oder Tripropylenglykol, Polyethylenglykole, Methylcellosolve, Cellosolve, Morpholine, Dioxan, N,N-Dimethylformamid, Dimethylsulfoxid, Tetrahydrofuran, Phthalate, Adipate oder Ester.

Bei den oben erwähnten Salzen sollte es sich im Falle medizinischer Anwendungen um pharmazeutisch annehmbare Salze handeln. Eine Anwendung von nicht pharmazeutisch annehmbaren Salzen ist dahingehend denkbar, sofern aus derartigen Salzen pharmazeutisch annehmbare Salze hergestellt werden können.

Geeignete Puffer im Sinne der vorliegenden Erfindung umfassen, sind jedoch nicht beschränkt auf, Essigsäure, Zitronensäure, Weinsäure, Borsäure oder Phosphorsäure in Kombination mit ihren korrespondierenden Base.

Geeignete Konservierungsstoffe im Sinne der vorliegenden Erfindung umfassen, sind jedoch nicht beschränkt auf, Benzalkoniumchlorid, Chlorbutanol, Thiomersal oder Parabene.

Erfindungsgemäß dienen die in vorliegender Anmeldung beschriebenen Zusammensetzungen der Vorbeugung und/oder Behandlung von Entzündungen oder Allergien der Atemwege, d. h. der oberen und/oder unteren Atemwege. In einer bevorzugten Ausführungsform werden die erfindungsgemäßen Zusammensetzungen zur Vorbeugung und/oder Behandlung von Entzündungen oder Allergien der oberen Atemwege eingesetzt.

Der hier verwendete Ausdruck "Behandlung" bezeichnet eine therapeutische Behandlung, in welcher dem Empfänger eine zur Vorbeugung, Linderung oder Beseitigung der Entzündung oder Allgergie effektive Menge der erfindungsgemäßen Zusammensetzungen verabreicht wird.

Der Begriff "obere Atemwege", wie er im Rahmen der vorliegenden Anmeldung verwendet wird, umfasst die Atemwege vom Eintritt der Luft in die Nase und/oder den Mund eines Individuums bis zum Beginn der Luftröhre, d. h. Nase, Mund, Rachen und Kehlkopf. Demgegenüber werden unter dem Begriff "untere Atemwege" definitionsgemäß Luftröhre, Bronchien, Bronchiolen und Lungenbläschen verstanden.

Entzündungen der Atemwege, welche mittels der erfindungsgemäßen Zusammensetzungen behandelbar sind, können durch Allergene, Mikroorganismen und/oder physikalische Reize hervorgerufen sein. Bevorzugt wird die Entzündung der Atemwege durch Mikroorganismen und/oder physikalische Reize hervorgerufen, wobei Mikroorganismen als Auslöser der Entzündung als besonders bevorzugt zu betrachten sind.

In einer bevorzugten Ausführungsform handelt es sich bei den die Entzündung hervorrufenden Allergenen um Fremdallergene, und stärker bevorzugt um Inhalationsallergene, Ingestionsallergene, Kontaktallergene, Injektionsallergene, Invasionsallergene und/oder Depotallergene. Alternativ kann die Entzündung der Atemwege auch durch Autoallergene hervorgerufen sein.

Inhalationsallergene umfassen, sind jedoch nicht beschränkt auf, Pflanzen- und Gräserpollen, Schimmelpilzsporen, Hausstaubmilbenkot, Tierhaare, Getreide- und Chemikalienstaub, oder Lösungsmitteldämpfe. Ingestionsallergene umfassen, sind jedoch nicht beschränkt auf, Nahrungsmittel (z.B. Milch, Fisch, Obst, Getreide, Nüsse) oder oral einzunehmende Medikamente. Kontaktallergene umfassen, sind jedoch nicht beschränkt auf, Chemikalien, synthetische Stoffe (z.B. Kunststoffe, Desinfektionsmittel, Medikamente), Metalle, Stoffe tierischen Ursprungs (z.B. Wolle, Seide), oder Stoffe pflanzlicher Herkunft (z.B. Harze). Injektionsallergene umfassen, sind jedoch nicht beschränkt auf, Medikamente, Vakzine, Impfseren sowie Insektengift. Invasions- und Depotallergene umfassen, sind jedoch nicht beschränkt auf, Operationsimplantate oder zahnärztliche Wurzelfüllmaterialien.

Der Ausdruck "Mikroorganismen" im Sinne der vorliegenden Erfindung umfasst Bakterien, Pilze, Hefen, Protozoen, Algen und Viren. Bevorzugt handelt es sich bei den die Entzündung hervorrufenden Mikroorganismen um Bakterien, Pilze oder Viren.

Eine Entzündung der Atemwege kann weiterhin durch physikalische Reize hervorgerufen sein, welche mechanischer, thermischer und/oder chemischer Natur sind. Mechanische Reize umfassen, sind jedoch nicht beschränkt auf, Druck, Verletzungen, Fremdkörpereinwirkung, oder dergleichen. Chemische Reize umfassen, sind jedoch nicht beschränkt auf, Säuren, Laugen, Toxine, entgleiste Enzyme, oder dergleichen. Wärme und Kälte stellen thermische Reize dar, welche Entzündungen der Atemwege hervorrufen können.

Entzündungen der Atemwege, welche mittels der erfindungsgemäßen pharmazeutischen Zusammensetzungen behandelt werden können, umfassen, sind jedoch nicht beschränkt auf, Rhinitis, Sinusitis und/oder Tracheobronchitis. In einer bevorzugten Ausführungsform handelt es sich bei der zu behandelnden Entzündung um Rhinitis.

Die vorliegende Erfindung betrifft weiterhin ein Medizinprodukt, welches Zinkcysteinat als Wirkstoff umfasst und als Spray, insbesondere als Nasenspray, formuliert ist.

## Patentansprüche

1. Verwendung von Zinkcysteinat zur Herstellung einer pharmazeutischen Zusammensetzung zur Behandlung von Entzündungen oder Allergien der Atemwege.

2. Verwendung nach Anspruch 1, **dadurch gekennzeichnet, dass** die pharmazeutische Zusammensetzung als Spray formuliert ist.

3. Verwendung nach Anspruch 1 oder 2, **dadurch gekennzeichnet, dass** die pharmazeutische Zusammensetzung zur nasalen und/oder oralen Verabreichung formuliert ist.

4. Verwendung nach einem der Ansprüche 1-3, **dadurch gekennzeichnet, dass** die pharmazeutische Zusammensetzung Zink in einer Menge im Bereich von 7 mg bis 10 mg pro Verabreichungsdosis bereitstellt.

5. Verwendung nach einem der Ansprüche 1-4, **dadurch gekennzeichnet, dass** die pharmazeutische Zusammensetzung weiterhin pharmazeutisch annehmbare Träger, Verdünnungsmittel und/oder Additive umfasst.

6. Verwendung nach Anspruch 5, **dadurch gekennzeichnet, dass** der pharmazeutisch annehmbare Träger Wasser, eine wässrige Salzlösung, ein nicht-wässriges Lösungsmittel, oder Mischungen hieraus umfasst.

7. Verwendung nach Anspruch 6, **dadurch gekennzeichnet, dass** die wässrige Salzlösung eine physiologische Kochsalzlösung ist.

8. Verwendung nach einem der Ansprüche 1-7, **dadurch gekennzeichnet, dass** die Entzündung durch Allergene, Mikroorganismen und/oder physikalische Reize hervorgerufen wird.

9. Verwendung nach Anspruch 8, **dadurch gekennzeichnet, dass** es sich bei den Allergenen um Fremdallergene und/oder Autoallergene handelt.

10. Verwendung nach Anspruch 9, **dadurch gekennzeichnet, dass** es sich bei den Fremdallergenen um Inhalationsallergene, Ingestionsallergene, Kontaktallergene, Injektionsallergene, Invasionsallergene und/oder Depotallergene handelt.

11. Verwendung nach Anspruch 8, **dadurch gekennzeichnet, dass** es sich bei den Mikroorganismen um Bakterien, Pilze und/oder Viren handelt.

12. Verwendung nach Anspruch 8, **dadurch gekennzeichnet, dass** die physikalischen Reize mechanischer, thermischer und/oder chemischer Natur sind.

13. Verwendung nach einem der Ansprüche 1-12, **dadurch gekennzeichnet, dass** es sich bei der Entzündung der Atemwege um Rhinitis handelt.

14. Verwendung von Zinkcysteinat zur Behandlung von Entzündungen oder Allergien der Atemwege.

15. Verfahren zur Behandlung von Entzündungen oder Allergien der Atemwege, **dadurch gekennzeichnet, dass** einem Patienten eine pharmazeutisch wirksame Menge an Zinkcysteinat verabreicht wird.

16. Medizinprodukt umfassend Zinkcysteinat als Wirkstoff, **dadurch gekennzeichnet, dass** das Medizinprodukt als Spray formuliert ist.
